# NEUE EUROPÄISCHE PATENTSCHRIFT

(11) **EP 1 881 858 B2**
(45) Veröffentlichungstag und Bekanntmachung des Hinweises auf die Entscheidung über den Einspruch: **27.05.2026**
(45) Hinweis auf die Patenterteilung: 23.09.2020
(21) Anmeldenummer: 06755150.7
(22) Anmeldetag: 11.05.2006
(51) Int. Cl.: A61M 5/168, A61M 5/142

(54) **VORRICHTUNG ZUR STEUERUNG EINER MEHRZAHL VON INFUSIONSPUMPEN**
DEVICE FOR CONTROLLING SEVERAL INFUSION PUMPS
DISPOSITIF POUR PILOTER UNE PLURALITE DE POMPES A PERFUSION

(30) Priorität: 14.05.2005 DE 102005022428
(43) Veröffentlichungstag der Anmeldung: 30.01.2008
(73) Patentinhaber: B. Braun Melsungen AG, 34212 Melsungen (DE)
(72) Erfinder: STEINKOGLER, Alexander, 81249 München (DE)
(74) Vertreter: Winter, Brandl - Partnerschaft mbB
(86) Internationale Anmeldenummer: PCT/EP2006/062238
(87) Internationale Veröffentlichungsnummer: WO 2006/122903

(56) Entgegenhaltungen:
- EP-A2- 0 906 767
- EP-A2- 0 960 627
- WO-A2-03/038566
- DE-A1- 19 823 240
- JP-A- 2001 212 230
- JP-A- 2004 097 757
- US-A- 4 741 732
- US-A1- 2005 085 760

## Beschreibung

Die Erfindung betrifft eine Vorrichtung zur Steuerung einer Mehrzahl von Infusionspumpen, wobei jeder Infusionspumpe jeweils ein Infusionsstoff zugeordnet ist, der innerhalb einer ihm zugeordneten vorbestimmbaren Zeitspanne als Infusion mit einer vorbestimmbaren Infusionsrate einem Lebewesen zugeführt wird, gemäß den Oberbegriffen der Patentansprüche 1 und 14.

In Krankenhäusern und anderen medizinischen Einrichtungen ist es üblich, Medikamente beziehungsweise Arzneiwirkstoffe, Vitamine, Nährstoffe, Drogen, Enzyme, Stoffwechselprodukte und dergleichen an kritisch kranke Patienten mittels sogenannter Infusionspumpen intravenös, dass heißt direkt in das Blut, in das Körpergewebe, den Verdauungstrakt, das respiratorische System, auf Schleimhäute oder die Haut zu übertragen. Bei Infusionspumpen sind im Wesentlichen als zwei Bauformen marktüblich erhältlich. Zum Einen handelt es sich dabei um Spritzenpumpen, die einen Spritzenkolben mit kontrollierter Vorschubgeschwindigkeit bewegen und damit den Spritzeninhalt intravenös verabreichen. Zum Anderen sind Pumpen gebräuchlich, die dem Patienten eine auf höheren Niveau in einer Infusionsflasche oder einem Infusionsbeutel befindliche Infusionsflüssigkeit mit kontrollierter Förderrate verabreichen.

Da sich im modernen Klinikbereich durchgesetzt hat, dass Medikamente beziehungsweise Arzneiwirkstoffe nicht mehr manuell mittels Spritze verabreicht werden, sondern voraussehbare Medikamentengaben für Infusionspumpen aufbereitet und von diesen den Patienten zugeführt werden, ist eine hohe Anforderung an die Genauigkeit und Zuverlässigkeit der Funktionsweise derartiger Infusionspumpen zwingende Voraussetzung für die Durchführung einer erfolgreichen Therapie. Die Zuführung von Medikamenten wird häufig mit konstanter Förderrate oder auch diskontinuierlich beziehungsweise mit Hilfe eines Förderratenprofils mit variabler Förderrate durchgeführt. Hierbei werden Systeme beziehungsweise Vorrichtungen mit einer Mehrzahl von Infusionspumpen verwendet, deren Anzahl bei einem typischen Intensivpflegeplatz zehn und mehr beträgt. Bei der Intensivpflege kritisch kranker Patienten, wie beispielsweise bei der Therapie von Patienten mit Herzerkrankungen, sind sogar bis zu zwanzig und mehr Infusionspumpen im Einsatz.

Bisher werden derartige Infusionspumpen als Einzelgeräte in ihrer Funktionsweise gesteuert und vorprogrammiert. Es wird also jede Infusionspumpe einzeln programmiert, um beispielsweise zu einem bestimmten Zeitpunkt die Infusion eines Infusionsstoffes, der in dieser Infusionspumpe enthalten ist, zu aktivieren oder zu deaktivieren. Dies erfordert eine getrennte Bedienung und Betätigung der einzelnen Infusionspumpen. Zudem wird hierdurch die gegenseitige Beeinflussung einzelner Medikamente, welche als Infusionsstoffe auf verschiedene Infusionspumpen verteilt sind, bei deren Verabreichung an den Patienten nicht berücksichtigt, sofern dies nicht durch den behandelnden Arzt beziehungsweise das Pflegepersonal selbst durchgeführt wird.

Eine automatische beziehungsweise selbsttätige Berücksichtigung der Auswirkung der Infusion mit einer vorbestimmbaren Infusionsrate und einer Verabreichung über eine bestimmte Zeitspanne kann herkömmlicherweise lediglich durch die einzelne Infusionspumpe selbst für den ihr zugeordneten Infusionsstoff durchgeführt werden. Beispielsweise würden physiologische Daten des Patienten zur laufenden Überprüfung einer Auswirkung des Infusionsstoffes auf den Patienten in jeder Infusionspumpe einzeln empfangen beziehungsweise eingegeben werden müssen, um eine auf diese Überprüfungsdaten abgestimmte neue Zeitspanne, Aktivierung oder Deaktivierung einer Infusion oder Unterbrechung derjenigen gezielt für diesen einen Infusionsstoff automatisch neu zu berechnen und dem Patienten zuzuführen. Hierbei findet keine Berücksichtigung der Wirkungen von Infusionsstoffen weiterer Infusionspumpen, die an der Therapie beteiligt sind, statt.

Bisher wird zur Betäubung von Patienten beispielsweise Propofol als Infusionsstoff in zwei Zeitabschnitten verabreicht, um einerseits den durch das Medikament verursachten Infusionsschmerz aufgrund einer niedrigen Konzentration zu lindern und andererseits eine möglichst langanhaltende Betäubung des Patienten ohne einen notwendigen Spritzen- oder Beutelwechsel durch eine möglichst hohe Medikamentenkonzentration zu erhalten. Hierfür wird im Allgemeinen das Propofol derart verdünnt, dass es 1%-ig innerhalb eines ersten Zeitabschnittes verabreicht wird, um innerhalb dieses Zeitabschnittes den Infusionsschmerz zu lindern. Sobald der Patient das Bewusstsein verloren hat, wird in einem weiteren Zeitabschnitt eine 2%-ige Propofollösung dem Patienten zugeführt. Eine derartige Umstellung von der 1- auf die 2-%ige Propofollösung wird durch das Bedienungspersonal beziehungsweise dem Benutzer der Infusionspumpe durchgeführt. Diese durch einen Menschen durchgeführte Umstellung bürgt - insbesondere im hektischen Klinikalltag - die Gefahr einer fehlerhaften oder zeitlich nicht rechtzeitigen Umstellung und somit eines Gesundheitsrisikos für den Patienten. Dies trifft insbesondere bei der Anwendung von pharmakokinetischen Modellen, die Grundlage für Injektionstrategien sind, zu, da den Modellen zugrundeliegenden Algorithmen abweichend von den bei einer fehlerhaften Handhabung tatsächlich verabreichten Propofolmengen Konzentrationswerte vorschreiben, welche die Schlussfolgerung einer Über- und Unterförderung des Infusionsstoffes beziehungsweise des zugeführten Medikamentes zwingend ergeben. Somit stellt die Umstellung von der einen auf die andere Propofollösung durch die Verwendung eines ersten Beutels bzw. Spritze mit der 1%-igen Propofollösung auf einen zweiten Beutel bzw. eine zweite Spritze mit der 2%-igen Propofollösung innerhalb einer Infusionspumpe ein Sicherheitsrisiko für den Patienten dar, da beispielsweise die Gefahr der zu kurzen Verabreichung der 1%-igen Propofollösung durch das Bedienpersonal zu einem Infusionsschmerz des Patienten führen kann, der noch nicht ausreichend betäubt ist. Darüber hinaus kann eine zu kurze Verabreichung der 1%igen Propofollösung unmittelbar zu einer zu langen Verabreichung der 2%igen Propofollösung und damit zu einer Überdosierung mit allen damit verbundenen Gefahren führen

Ebenso stellt die Verwendung von zwei unabhängig voneinander funktionierenden Infusionspumpen mit zwei unterschiedlich verdünnten Propofollösungen während der Umstellung von der einen auf die andere Propofollösung ein Sicherheitsrisiko für den Patienten dar, da beispielsweise entweder die niedrig konzentrierte Propofollösung zu kurz verabreicht wird und der Patient zunächst einen Infusionsschmerz erleidet und anschliessend eine Überdosierung des Medikamentes erfährt, oder die hochkonzentrierte Propofollösung zu kurz zugeführt wird, woraus sich eine unzureichende Betäubung des Patienten ergibt.

Aus der Druckschrift US 2005/0085760A1 ist ein medizinisches Fluidflusssystem mit einer Steuervorrichtung bekannt, die Fluidpumpen innerhalb einer zentralen Fluidflussmaschine mit ausgelagerten Fluidpumpen in beispielsweise Infusionsvorrichtungen koordiniert. Die zentrale Steuervorrichtung verringert den Zeitaufwand bei der Einstellung von Therapieparametern, da mehrere Pumpen zentral gesteuert werden können, erhöht allerdings auch die Fehleranfälligkeit des Systems.

Die Druckschrift EP 0 906 767 A2 befasst sich mit einem iontophoretischen Medikamentenapplikationssystem, bei dem mehrere Elektroden entweder zentral nach Art einer Master- Slave-Hierarchie gesteuert werden, oder im speziellen Fall, dass eine Medikamentendosis aufgeteilt auf mehrere Elektroden verabreicht werden muss, mehrere Elektroden direkt miteinander kommunizieren. In diesem speziellen Fall wird die Gesamtdosis stets durch die Anzahl der Elektroden geteilt, um zu verhindern, dass einem Patienten aufgrund hoher iontophoretischer Spannungen Schmerz zugefügt wird. Eine derartige Problematik tritt allerdings nur bei iontophoretischen Verfahren auf.

Aus der Druckschrift WO 03/038566 A2 ist eine Benutzerschnittstelle für Verabreichungsgeräte von Sedativa und Analgetika bekannt. Diese Benutzerschnittstelle ist beispielsweise ein Touch Screen, auf welchem ein Behandlungsverlauf angezeigt wird.

Weiterhin ist aus der Druckschrift EP 0 960 627 A2 eine Vorrichtung zur zentralen Steuerung und Überwachung von mehreren Infusionspumpen bekannt. Eine solche zentrale Steuerungsvorrichtung vereinfacht die Weiterleitung von Behandlungsdaten an Patientenmanagementsysteme und Alarmsysteme, erhöht allerdings die Fehleranfälligkeit der Steuerung der Infusionspumpen.

Aus DE 38 17411 C2 ist eine Vorrichtung zur konzentrierten, kontinuierlichen und gleichzeitigen Infusion von mehreren Infusionslösungen bekannt, wobei mittels Förderleitungen die Infusionslösungen über ein Übergangsstück mit einer gemeinsamen Patientenleitung dem Patienten verabreicht werden. Somit findet eine zeitgleiche Förderung der Infusionslösungen und eine vor deren Verabreichung durchgeführte Vermengung dieser Infusionslösungen statt. Mittels einer zentralen Steuereinheit werden die Förderraten der Infusionspumpen geregelt. Eine gegenseitige Berücksichtigung der Infusionspumpen hinsichtlich ihrer Infusions- rate und den Förderungszeitpunkten in Abhängigkeit von deren tatsächlichen Werten findet nicht statt. Es wird allenfalls mittels eines in der zentralen Steuereinheit abgespeicherten Programms die Förderrate der einzelnen Infusionspumpen vorbestimmt.

Demzufolge liegt der vorliegenden Erfindung die Aufgabe zugrunde, eine Vorrichtung zur Steuerung einer Mehrzahl von Infusionspumpen zur Verfügung zu stellen, welches/welche das Risiko einer Fehleingabe minimiert, einen geringen Bedienungsaufwand erfordert und einfach in der Handhabung ist.

Diese Aufgabe wird durch die Merkmale des Patentanspruches 1 gelöst.

Ein wesentlicher Punkt der Erfindung liegt darin, dass bei einer Vorrichtung zur Steuerung einer Mehrzahl von Infusionspumpen, wobei jeder Infusionspumpe jeweils ein Infusionsstoff zugeordnet ist, der innerhalb einer ihm zugeordneten vorbestimmbaren Zeitspanne als Infusion mit einer vorbestimmbaren Infusionsrate einem Lebewesen zu- geführt wird, die Infusionspumpen selbsttätig Steuerungsdaten zum zeitlich aufeinander abgestimmten Aktivieren und Deaktivieren der verschiedenen Infusionen austauschen. Durch ein derartiges Verfahren wird eine gegenseitig zeitliche Abhängigkeit der Aktivierung beziehungsweise Deaktivierung einzelner Infusionspumpen innerhalb einer Infusionspumpenvorrichtung erreicht, die eine getrennte Bedienung jeder einzelnen Infusions- pumpe zum richtigen Zeitpunkt einer durchzuführenden Therapie erübrigt. Somit wird das Risiko einer fehlerhaften Eingabe bedingt durch menschliches Versagen und damit das Ge- sundheitsrisiko des zu behandelnden Patienten weitestgehend ausgeschaltet. Vielmehr fin- det eine automatische Übertragung von Steuerungsdaten mittels die einzelnen Infusions- pumpen verbindenden Datenleitungen an eine oder mehrere vorausgewählte Infusionspum- pen statt, sobald die Zeitspanne der Zuführung einer ersten Infusion dieser sendenden Infusionspumpe beendet ist und eine zweite Infusion aus der die Steuerungsdaten empfangenden weiteren Infusionspumpe durch deren Aktivierung dem Patienten verabreicht werden soll.

Ebenso ist die zeitlich überschneidende gleichzeitige Infusion mehrerer Infusionsstoffe aus verschiedenen Infusionspumpen denkbar, wobei Start- und Endzeitpunkt der Zeitspanne, innerhalb welcher die verschiedenen Infusionen dem Patienten zugeführt werden, durch selbständigen Datenaustausch der Steuerungsdaten zwischen den einzelnen Infusionspumpen ohne Zwischenschaltung von Bedienungspersonal gegebenenfalls anhand eines vorgegebenen Programms oder einer vorausgegangenen Eingabe eines Steuervorganges, der auf einem pharmakokinetischen Modell beruht, festgelegt werden.

Ebenso können zwei verschiedene Medikamente beziehungsweise Arzneiwirkstoffe aus zwei verschiedenen Infusionspumpen abwechselnd, jedoch zeitlich aufeinander abgestimmt dem Patienten verabreicht werden. Eine auf einen derartigen Steuerungsvorgang basierende Therapie ermöglicht eine verlängerte Verabreichung dieser beiden Medikamente, ohne dass der Eingriff von Klinikpersonal zum Bedienen der Infusionspumpen erfolgen muss.

Durch die stattfindende Kommunikation zwischen den Infusionspumpen wird es ermöglicht, dass eine chemische Beeinflussung bzw. eine Beeinflussung der Wirkung der Medikamente, welche auf die verschiedenen Infusionspumpen aufgeteilt sind - gegebenenfalls unter Berücksichtigung eines Patientenmodells - derart berücksichtigt werden, dass eine der Infusionspumpen ihre Auswahl bzw. die Auswahl weiterer Infusionspumpen verweigert, sofern festgestellt wird, dass die gemeinsame Zuführung derjenigen Medikamente, die diesen Infusionspumpen als Infusionspumpen als Infusionsstoffe zugeordnet sind, eine negative chemische Reaktion, wie beispielsweise eine Flockung des Gesamtgemisches, oder eine negative Beeinflussung ihrer Wirkung auf den Patienten zur Folge hätte. Ebenso kann eine Infusionspumpe die Kombination mit anderen Infusionspumpen bzw. deren zugeordneten Medikamenten vorschlagen bzw. in der Auswahl durch ein Bedienungspersonal zulassen, wenn hierdurch eine maximal positive Beeinflussung sowohl hinsichtlich ihrer Wirkung als auch hinsichtlich ihrer chemischen Reaktion erreicht wird.

Zusätzlich zu einer mittels Infusion durchgeführten Therapie oder alternativ hierzu kann mittels einer weiteren Infusionspumpe beispielsweise eine Kochsalzlösung zur Erfüllung einer niedrigeren Rate der KVO-Rate zugeführt werden, während keine Arzneiwirkstoffe injiziert werden. Die KVO-Rate soll sicher stellen, dass die Vene und damit der Zugang zum Patienten nicht verschlossen wird, wenn keine Flüssigkeit durch den Zugang injiziert wird.

Die Steuerungsdaten werden entweder mittels einer jeder Infusionspumpe zugeordneten Steuereinheit, die vorzugsweise in der Infusionspumpe integriert ist, direkt zwischen den Infusionspumpen ausgetauscht und innerhalb der einzelnen Infusionspumpen verarbeitet oder mittels einer zentralen Steuereinrichtung, über welche die Kommunikation beziehungsweise der Datenaustausch zwischen den einzelnen Infusionspumpen übertragen, weitergeleitet und gegebenenfalls beeinflusst wird, ausgetauscht. Hierfür weisen die einzelnen Steuereinheiten oder die zentrale Steuereinrichtung Betätigungselemente auf, um mindestens einen Steuervorgang zur Steuerung der Aktivierung und Deaktivierung der verschiedenen Infusionen für mindestens zwei Infusionspumpen einzugeben. Vorzugsweise bewirkt ein derartiger Steuervorgang eine gleichzeitige Aktivierung und/oder Deaktivierung und/oder eine Unterbrechung der Infusionen der mindestens zwei Infusionspumpen.

Gemäß einer bevorzugten Ausführungsform kann eine derartige Aktivierung und/oder Deaktivierung der Infusionen mittels eines gemeinsamen Startschaltelementes begonnen und mittels eines gemeinsamen Stoppschaltelementes beendet werden. Alternativ kann durch mehrmaliges Drucken des Startschaltelementes eine Nacheinanderaktivierung der verschiedenen Infusionspumpen erfolgen. Andersherum kann durch ein mehrmaliges Drücken des Stoppschaltelementes eine Deaktivierung der einzelnen Infusionspumpen in einer vorbestimmbaren Reihenfolge erfolgen.

Der Steuervorgang kann einer vom Bedienungspersonal ausgewählten Therapie, die beispielsweise mittels eines Barcode-Scan-Vorganges festgelegt wird, zugeordnet sein, wobei selbsttätig diejenigen Infusionspumpen, welche dem Therapievorgang aufgrund des darin enthaltenen Infusionsstoffes zugeordnet werden können, von den Steuereinheiten oder der zentralen Steuereinrichtung automatisch ausgewählt oder dem Benutzer zur Auswahl angezeigt werden. Hierdurch wird eine automatische Zuweisung derjenigen Infusionspumpen, welche die für die Therapie gewünschten Infusionsstoffe bereits enthalten, durchgeführt, wobei dies in Abhängigkeit von der beabsichtigten Infusionsrate und der in den Infusionspumpen enthaltenden Mengen der Infusionsstoffe sowie sonstigen Parametern der einzelnen Infusionspumpen, wie beispielsweise deren maximal mögliche Förderungsrate, geschehen kann.

Vorzugsweise führt eine der Infusionspumpe dem Lebewesen ein Verdünnungsmittel zu einem Arzneiwirkstoff, der mittels einer weiteren Infusionspumpe zugeführt wird, zu. Hierbei können die ausgetauschten Steuerungsdaten eine Deaktivierung der Infusion des Verdünnungsmittels unter Beibehaltung der Aktivierung des Arzneiwirkstoffes bewirken. Auf diese Weise wird es ermöglicht, dass durch selbsttätige Abschaltung derjenigen Infusionspumpe, die das Verdünnungsmittel zu einem Betäubungsmittel, wie beispielsweise Propofol, dem Patienten zuführt, eine Erhöhung der Konzentration und des Betäubungsmittels automatisch bewirkt wird, ohne dass hierzu eine Handhabung durch das Bedienungspersonal erforderlich ist. Alternativ kann durch einen kurzen Knopfdruck des Bedienungspersonals eine Abschaltung der das Verdünnungsmittel enthaltenen Infusionspumpe bewirkt werden, nachdem das Bedienungspersonal festgestellt hat, dass der Patient bereits das Bewusstsein verloren hat und aufgrund des nicht mehr vorhandenen Infusionsschmerzes eine höhere resultierende Konzentration des Betäubungsmittels möglich ist. Hierdurch wird eine Verwechslung der Konzentrationen des Betäubungsmittels in zwei verschiedenen Zeitspannen bei der Verwendung von pharmakokinetischen Modellen vermieden.

Gemessene oder geschätzte physiologische Daten des Patienten können in die Steuereinheiten oder die zentrale Steuereinrichtung eingegeben beziehungsweise während einer Therapie laufend empfangen werden, um die Infusionsraten, die Zeitspannen der einzelnen Infusionen, die Zeitpunkte der Aktivierung und Deaktivierung und gegebenenfalls weitere Infusionsparameter selbsttätig mittels vorbestimmter Programmschritte - auch durch Datenübertragung der Steuerungsdaten an beteiligte Infusionspumpen - zu verändern.

Eine Infusionsvorrichtung zur Durchführung eines derartigen Verfahrens weist vorteilhaft neben den Steuereinheiten innerhalb der einzelnen Infusionspumpen und/oder gegebenenfalls einer zentralen Steuereinrichtung sowie den Betätigungselementen eine Anzeigeeinrichtung entweder an jeder Infusionspumpe oder als zentrale Anzeigeeinrichtung auf, um die Steuerungsdaten, die gemessenen physiologischen Daten des Lebewesens, die Zeitpunkte der Aktivierung und Deaktivierung der Infusionspumpen, die Zeitspannen der Infusionen und/oder der Infusionsrate anzuzeigen. Dies ermöglicht eine laufende Kontrolle des momentanen Therapiezustandes des Patienten durch das Bedienungspersonal, um gegebenenfalls einen vorzeitigen Abbruch oder eine Verlängerung der Therapie beziehungsweise eine Veränderung der Therapieparameter durchzuführen.

Vorteilhaft wird insbesondere bei der Anwendung der zentralen Steuereinrichtung die Anzeigeeinrichtung zur Anzeige für die in der Infusionsvorrichtung vorhandenen Infusionspumpen mit der Steuereinrichtung gekoppelt bzw. in dieser integriert. Dadurch können die Förderraten mehrerer an einer Therapie beteiligter Infusionspumpen gleichzeitig, beispielsweise in einem gemeinsamen Diagramm dargestellt werden. Die Steuereinrichtung kann zudem mittels der Anzeigeeinrichtung die gemessenen und/oder geschätzten physiologischen Daten des Patienten zusätzlich oder alternativ zu den Förderraten der einzelnen Infusionspumpen wiedergeben. Hierdurch ergibt sich eine zusammengefasste abstrakte Darstellung, die sich mehr auf die Wirkung einer Therapie als auf ihre Förderraten bezieht.

Ebenso kann eine Alarmierung für die einzelnen Infusionspumpen an der zentralen Steuereinrichtung derart vorgenommen werden, dass ein Alarm bei bevorstehender Entleerung einer Infusionspumpe ausgelöst wird. Vorteilhaft kann es erfindungsgemäß durch den Austausch von Daten zwischen den Infusionspumpen zu einer Reduktion unnötiger Alarme kommen, wenn beispielsweise eine Infusionspumpe bei ihrer derzeitigen Förderrate zwar bald leer werden würde, dieser Fall aber aufgrund des bereits bekannten Infusionsverhaltens einer weiteren im System befindlichen Infusionspumpe nicht auftreten wird.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1: in einer schematischen Darstellung eine beispielhafte Anordnung einer Infusionsvorrichtung mit einer Mehrzahl von Infusionspumpen gemäß einer Ausführungsform der Erfindung;
- Fig. 2: in einer schematischen Darstellung ein Flussdiagramm zu dem Ablauf eines erfindungsgemäßen Verfahrens;
- Fig. 3: in einer schematischen Darstellung das vorrichtungsmäßig dargestellte erfindungsgemäße Verfahren gemäß einer Ausführungsform der Erfindung; und
- Fig. 4: in einer schematischen Darstellung das vorrichtungsmäßig dargestellte Verfahren gemäß einer weiteren Ausführungsform der Erfindung.

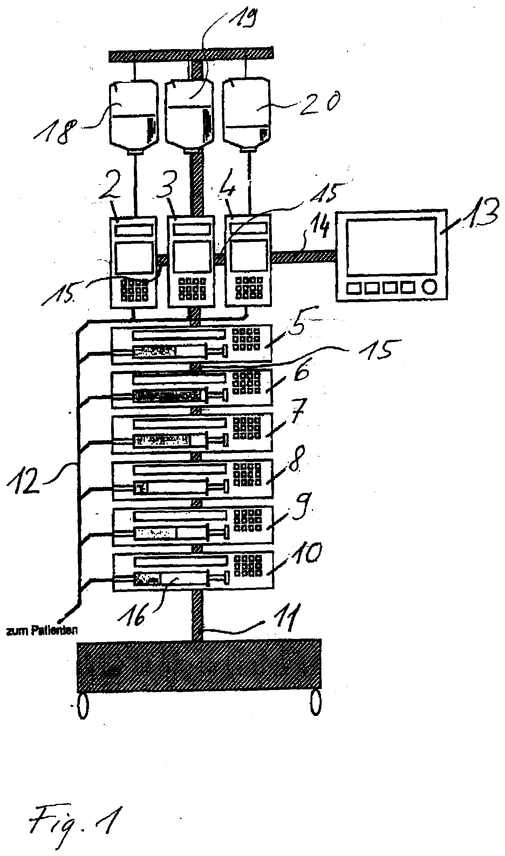

In Fig. 1 wird in einer schematischen Darstellung die Anordnung einer Infusionspumpenvorrichtung gemäß einer Ausführungsform der Erfindung gezeigt. Die Infusionspumpenvorrichtung 1 umfasst die Infusionspumpen 2-10, wobei es sich bei den Infusionspumpen 5-10 um Spritzenpumpen handelt.

Die Infusionspumpen sind auf einer gemeinsamen Säule 11 eines Infusionspumpenträgers angeordnet. Die räumliche Anordnung der einzelnen Infusionspumpen 2-10 auf einem derartigen Träger oder auch einem anders geformten Träger ist nahezu beliebig, sofern die einzelnen Infusionspumpen mittels Datenleitungen, die innerhalb der Säule 11 angeordnet sein können, miteinander verbunden sind. Eine derartige Verbindung kann sowohl reihen- als auch sternartig zur Übertragung der Steuerungsdaten an die jeweils weiteren Infusionspumpen ausgebildet sein.

Vorteilhaft kann anstelle einer Kabelverbindung eine kabellose Übertragung der Daten stattfinden. Dies kann beispielsweise mittels modulierten Ultraschallwellen, modulierten Lichtwellen (z.B. IrDa) oder mittels Funkwellen (beispielsweise blue tooth) erfolgen.

An einem Trägerabschnitt 14 ist alternativ oder zusätzlich zu einzelnen innerhalb der Infusionspumpen angeordneten Steuereinheiten welche hier nicht näher dargestellt sind, eine zentrale Steuereinrichtung 13 mit einer Anzeigeeinrichtung angeordnet. An dieser Steuer- und Anzeigeeinrichtung kann eine zentrale Programmierung, Bedienung und Kontrolle sämtlicher Infusionspumpen insbesondere hinsichtlich der zwischen ihnen ausgetauschten Steuerungsdaten zur Aktivierung und/oder Deaktivierung einzelner Infusionen dieser Infusionspumpen 2-10 durchgeführt werden.

Datenverbindungen, die innerhalb von Säulenabschnitten 15 zwischen den Infusionspumpen verlaufen, übertragen die Steuerungsdaten, die notwendig sind, um eine gegenseitige Abhängigkeit einer stattfindenden oder nicht stattfindenden Schiebebewegung von Spritzen 16 der Infusionspumpen zu regeln.

Für die zusätzliche Eingabe einzelner Parameter, wie beispielsweise physiologischer Parameter der Patienten oder eine Programmierung der innerhalb der Infusionspumpe enthaltenen Steuereinheit weist jede Infusionspumpe eine Tastatur 17 auf.

Zusätzlich sind Flüssigkeitsreservoirs 18, 19 und 20 vorhanden. Eine gemeinsame Infusionsleitung 12 führt das gemeinsame Infusionsgemisch dem Patienten zu.

Da sich die medizinischen Notwendigkeiten bei einem Intensivpatienten im Laufe seines Aufenthalts auf einer Intensivstation ändern, ist eine solche Infusionsvorrichtung mit einer Mehrzahl von Infusionspumpen als ein dynamisches System zu verstehen, bei dem Systemkomponenten im laufenden Betrieb ergänzt oder entfernt werden können und bei dem Betriebsparameter, wie zum Beispiel zu verabreichende Medikamente, zu verabreichende Dosis, zeitlicher Verlauf der Verabreichung und ähnliches im laufenden Betrieb verändert werden können. Aus diesem Grund ist eine automatische Erkennung physiologischer Daten des Patienten, die laufend gemessen werden, notwendig, welches durch eine hier nicht näher dargestellte Messdatenleitung vom Patienten zu den einzelnen Steuereinheiten oder der zentralen Steuereinrichtung geschehen kann.

Zusätzlich oder alternativ können pharmakokinetische Modelle innerhalb der Infusionsvorrichtung eingespeichert sein, wobei derartige Modelle die Verteilung eines verabreichten Medikaments im Körper des Patienten durch geeignete Modelldarstellung simulieren. Diese Modelle sind medikamentenspezifisch und erhalten weitere Eingabeparameter, wie Patientengewicht oder-alter. Die Zielgröße stellt dabei beispielsweise einen gewünschten Plasmaspiegel im Blut der Patienten dar, also eine gewünschte Konzentration dieses Medikaments im Blut. Diese Zielkonzentration kann zeitlich konstant sein, was nur den Ersatz der vom Körper pro Zeiteinheit resobierten Menge des Medikaments bedingt, oder sie kann zeitvariabel sein.

Aufgabe des pharmakokinetischen Modells ist es nun, durch einen Regelalgorithmus die Förderrate der einzelnen Infusionspumpen so zu steuern, dass unter Berücksichtung der Eingabeparameter die tatsächlich im Blut vorhandenen Konzentration dieses Medikaments gleich der Zielkonzentration ist. Ein solches pharmakokinetisches Modell kann sowohl in einer Steuereinheit einer jeden Infusionspumpe selbst als auch in der zentralen Steuereinrichtung 13 implementiert sein.

Durch die mittels austauschbarer Steuerungsdaten miteinander verbundenen Infusionspumpen kann ein bisher vom Arzt meist manuell aufgesetzter Verordnungsbogen, der eingetragene Medikamentenverordnungen enthält, wobei die Medikamente vom Pflegepersonal im Laufe eines Tages als Medikamentengaben verabreicht werden, zu den Steuereinheiten in Infusionspumpen automatisch übertragen werden, indem diese eine oder mehrere Schnittstellen innerhalb ihres Datenverbindungsnetzes aufweisen, um eine Verbindung zu einer übergeordneten Datenverarbeitungseinrichtung, in welcher der Verordnungsplan anstelle des manuell auszufüllenden Verordnungsbogens eingegeben wird, aufzubauen. Alternativ kann der Verordnungsplan direkt an der zentralen Steuerungs- bzw. Anzeigeeinrichtung oder in einer der Steuereinheiten der miteinander datentechnisch verbundenen Infusionspumpen eingegeben werden.

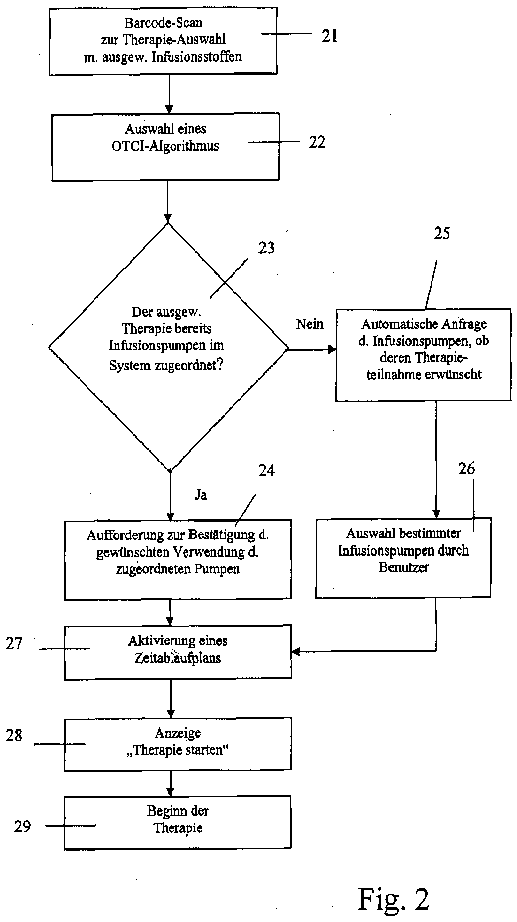

In Fig. 2 wird in einem schematischen Flussdiagramm der erfindungsgemäße Verfahrensablauf gemäß einer Ausführungsform der Erfindung gezeigt. Das Bedienungspersonal wird gemäß dem Schritt 21 von der Infusionsvorrichtung mittels der zentralen Anzeigeeinrichtung oder einer Anzeige in einer der Infusionspumpen dazu aufgefordert, einen Barcode-Scan zur Auswahl einer bestimmten Therapie durchzuführen. Hierfür wird ein an die Infusionspumpe externer angeschlossener Barcode-Scanner zum Einlesen von Barcodes, die einzelnen Therapien zugeordnet sind, verwendet.

Im vorliegenden Fall wird als ausgewählte Therapie eine Betäubung mittels Propofol und einem Verdünnungsmittel gewünscht. Sobald eine derartige Propofol-Verdünnungsmittel-Therapie ausgewählt ist, wird automatisch von der Infusionsvorrichtung beziehungsweise der einzelnen Infusionspumpe ein zugeöriger OTCI-Algorithmus für eine noch zu bestimmende Infusionspumpe festgelegt (Schritt 22).

In einem Schritt 23 wird automatisch festgestellt, ob der ausgewählten Therapie bereits Pumpen innerhalb der Infusionsvorrichtung beziehungsweise dem System zugeordnet sind. Dies kann beispielsweise davon abhängig gemacht werden, ob bereits eine Infusionspumpe mit Propofol und eine weitere Infusionspumpe mit dem zugehörigen Infusionsmittel gefüllt sind.

Sofern derartige Pumpen bereits vorhanden sind, wird das Bedienungspersonal in einem Schritt 24 dazu aufgefordert, diese Auswahl der zugeordneten Pumpen zu bestätigen.

Wenn keine automatische Zuordnung der Infusionspumpen in einem Schritt 23 erfolgt, findet in einem Schritt 25 eine automatische Anfrage der Steuereinheiten einzelner Infusionspumpen, die einen der beteiligten Infusionsstoffe zugewiesen bekommen, statt, ob sie die an der Therapie beteiligten Pumpen sein sollen. Sofern das Bedienungspersonal die angebotenen Pumpen gemäß Schritt 26 ausgewählt hat, findet die Aktivierung eines Zeitablaufplans gemäß Schritt 27 statt.

In einem derartigen Zeitablaufplan wird beispielsweise der gleichzeitige Start einer ersten Infusionspumpe mit Propofol als Infusionsstoff und einer zweiten Infusionspumpe mit dem Verdünnungsmittel, beispielsweise auf Fettbasis basierend, initiiert. Beide Infusionsstoffe werden miteinander vermischt dem Patienten zugeführt. Hierdurch ergibt sich eine Propofollösung mit einer Konzentration, die unterhalb von 1 % liegt. Dies ermöglicht die Zuführung des verdünnten Propofols mit geringem Injektionsschmerz.

Sobald der Patient das Bewusstsein verloren hat, können mittels des vorbestimmbaren Zeitplans Steuerungsdaten von der ersten Infusionspumpe zu der zweiten das Verdünnungsmittel enthaltenen Infusionspumpe übertragen werden, welche eine Abschaltung der zweiten Infusionspumpe bewirken, um Propofol in hoher Konzentration zur Aufrechterhaltung der Betäubung zuzuführen.

Sofern der betreuende Arzt beziehungsweise das Bedienungspersonal einen vorzeitigen Abbruch des Betäubungsvorganges wünscht, kann er dies durch Drücken einer Taste, die auf einer der beiden Infusionspumpen oder beiden angeordnet ist, erreichen, wodurch beide Infusionspumpen gleichzeitig aufgrund der Übertragung von Steuerungsdaten zwischen den Infusionspumpen abgeschaltet werden.

Sobald der Zeitablaufplan aktiviert ist, gibt die Infusionsvorrichtung oder die einzelne Infusionspumpe die Anzeige "Therapie starten" gemäß dem Schritt 28 frei. Anschließend beginnt die Therapie gemäß dem Schritt 29.

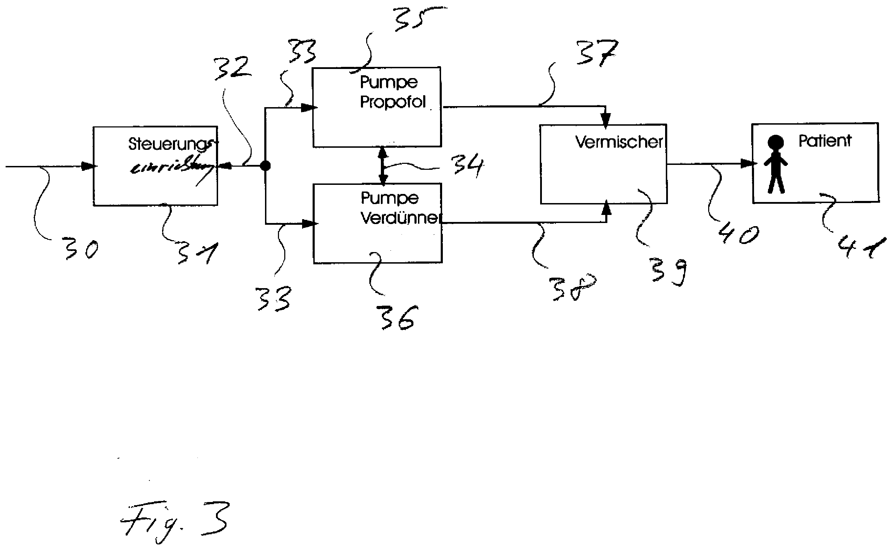

In Fig. 3 wird in einer schematischen Darstellung das erfindungsgemäße Verfahren anhand der Darstellung der Infusionsvorrichtung dargestellt. Durch Eingabe von Daten, wie es durch Pfeil 30 wiedergegeben wird, in eine zentrale Steuereinrichtung 31 findet eine Vorprogrammierung des gewünschten Verfahrensablaufes, in diesem Fall der Behandlung mit Propofol statt. Durch gegenseitigen Datenaustausch der Steuereinrichtung 31 mit einer Infusionspumpe 35, welche Propofol enthält, und einer Infusionspumpe 36, welche das Verdünnungsmittel zu dem Betäubungsmittel Propofol enthält, findet eine Kommunikation zwischen den Infusionspumpen 35, 36 und der zentralen Steuereinrichtung 31 und insbesondere zwischen den Infusionspumpen 35, 36 statt, wie es durch die Pfeile 32, 33 und 34 wiedergegeben wird. Hierdurch ist es möglich, dass mittels einer Programmierung und eines Datenaustausches zwischen den Infusionspumpen, wie es durch den Doppelpfeil 34 dargestellt ist, eine rechtzeitige Abschaltung des Verdünnungsmittels und damit der Infusionspumpe 36 stattfindet, wenn der Patient 41 sich im ausreichenden Betäubungszustand befindet.

Sowohl das Propofol als auch das Verdünnungsmittel werden bis zu diesem Zeitpunkt über Leitungen 37, 38 innerhalb eines Vermischers 39 vermischt und über eine gemeinsame Leitung 40 dem Patienten 41 zugeführt.

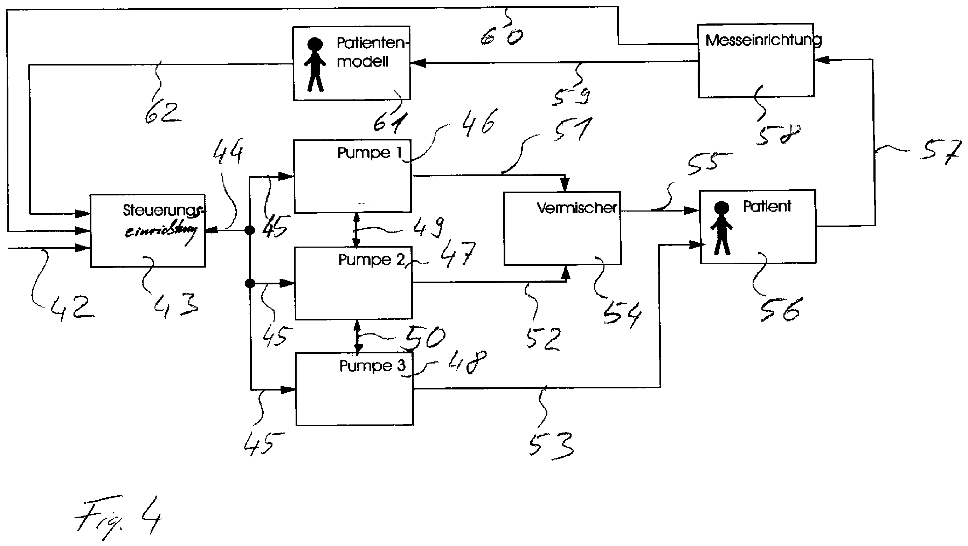

In Fig. 4 wird in einer schematischen Darstellung das erfindungsgemäße Verfahren anhand einer weiteren Infusionsvorrichtung wiedergegeben. Wiederum werden wie durch den Pfeil 42 dargestellt, Daten zur Programmierung einer Steuerungseinrichtung 43 eingegeben und mittels eines Datenaustausches zwischen der zentralen Steuereinrichtung 43 und einzelnen Infusionspumpen 46, 47 und 48 über Datenaustauschleitungen 44 und 45 an die Infusionspumpen weitergegeben, wobei die Infusionspumpen 46, 47, 48 diese Daten mit der zentralen Steuereinrichtung 43 beispielsweise zur geeigneten Auswahl bestimmter Infusionspumpen mittels der Leitungen 44 und 45 austauscht und insbesondere untereinander mittels Leitungen 49, 50 den Datenaustausch zur Überprüfung einer geeigneten Kombination derjenigen Infusionspumpen durchführt.

Über Leitungen 51, 52 werden Infusionsstoffe der Infusionspumpen 46, 47 in einem Vermischer 54 miteinander vermischt und über die Leitung 55 einem Patienten 56 ebenso wie über eine Leitung 53 aus der Infusionspumpe 48 zugeführt. Anschließend findet eine Messung der physiologischen Daten des Patienten mittels einer Messeinrichtung 58 und einer Leitung 57 statt.

Die gemessenen Daten des Patienten werden entweder direkt mittels einer Verbindung 60 oder indirekt über ein Patientenmodell 61 mittels Verbindungen 59, 62 zur Steuerungseinrichtung 43 rückgekoppelt. Hierbei findet ein Abgleich der gemessenen physiologischen Daten mit vorgegebenen Daten gemäß dem Patientenmodell 61 statt.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Infusionsvorrichtung
- 2-10: Infusionspumpen
- 11: Säule
- 12: Infusionsleitung
- 13: zentrale Steuereinrichtung
- 14, 15: Säulenabschnitt
- 16: Spritzen
- 17: Eingabeelemente
- 18, 19, 20: Flüssigkeitsbehälter
- 21: Schritt des Barcode-Scannens
- 22: Schritt der Auswahl der OTCI-Algoithmus
- 23: Schritt des Zuordnens von Pumpen
- 24: Schritt der Aufforderung zur Bestätigung der ausgewählten Pumpen
- 25: Schritt der automatischen Anfragen möglicher zuzuordnender Infusionspumpen
- 26: Schritt der Auswahl der möglich zuordbaren Infusionspumpen
- 27: Schritt des Aktivierens eines Zeitablaufplanes
- 28: Schritt des Anzeigens "Therapie starten"
- 29: Schritt der Beginn der Therapie
- 30,42: Dateneingabe
- 31, 43: zentrale Steuereinrichtung
- 32, 33, 34, 44, 45, 49, 50: Datenaustauschverbindungen
- 35, 36, 46, 47, 48: Infusionspumpen
- 37, 38, 40, 51, 52, 53, 55: Infusionsstoffleitungen
- 39, 54: Vermischer
- 41, 56: Patient
- 57, 59, 60: Messdatenübertragungsverbindungen
- 58: Messeinrichtung
- 61: Patientenmodell
- 62: Abgleich Daten aus Patientenmodell und gemessenen Daten

## Patentansprüche

1. Vorrichtung zur Steuerung einer Mehrzahl von Infusionspumpen (2-10), mit einem Infusionspumpenträger mit einer Säule (11), an welcher die Mehrzahl der Infusionspumpen angeordnet ist, wobei jeder Infusionspumpe (2-10) jeweils ein Infusionsstoff zugeordnet ist, der innerhalb einer ihm zugeordneten vorbestimmbaren Zeitspanne als Infusion mit einer vorbestimmbaren Infusionsrate einem Lebewesen zuführbar ist,
wobei die Vorrichtung eingerichtet ist, zuvor festgelegte Steuerungsdaten zur zeitlich aufeinander abgestimmten Aktivierung und Deaktivierung mittels Steuereinheiten selbsttätig zwischen den Infusionspumpen (2-10) ohne Zwischenschaltung von Bedienungspersonal auszutauschen, wobei jede Infusionspumpe (2-10) eine der Steuereinheiten aufweist, welche die Steuerdaten verarbeitet und an Steuereinheiten der weiteren Infusionspumpen (2-10) direkt überträgt, und
wobei die Vorrichtung derart eingerichtet ist, dass eine automatische Übertragung von Steuerungsdaten an eine oder mehrere vorausgewählte Infusionspumpen stattfindet, sobald die Zeitspanne der Zuführung einer ersten Infusion der die Steuerungsdaten sendenden Infusionspumpe beendet ist und eine zweite Infusion aus der die Steuerungsdaten empfangenden weiteren Infusionspumpe durch deren Aktivierung dem Patienten verabreicht werden soll,
**dadurch gekennzeichnet, dass**
die Vorrichtung derart eingerichtet ist, dass gemessene oder geschätzte physiologische Daten in die Steuereinheiten eingegeben und während einer Therapie laufend empfangen werden können, um Infusionsraten, die Zeitspannen von einzelnen Infusionen oder die Zeitpunkte der Aktivierung und Deaktivierung selbsttätig mittels vorbestimmter Programmschritte zu verändern.

2. Vorrichtung nach Anspruch 1, **gekennzeichnet durch** Betätigungselemente an den Infusionspumpen (2-10) zur Eingabe von Steuerbefehlen, zur Auswahl von Steuervorgängen, zur Auswahl von an den Steuervorgängen beteiligten Infusionspumpen (2-10) und/oder zur Eingabe von physiologischen Daten des Lebewesens.

3. Vorrichtung nach Anspruch 1 oder 2, **gekennzeichnet durch** eine Anzeigeeinrichtung an jeder Infusionspumpe und/oder eine zentrale Anzeigeeinrichtung zur Anzeige der Steuerungsdaten, gemessener oder geschätzter physiologischer Daten des Lebewesens, der Zeitpunkte der Aktivierung und Deaktivierung der Infusionspumpen, der Zeitspannen der Infusion und/oder der Infusionsraten.

4. Vorrichtung nach Anspruch 3, **dadurch gekennzeichnet, dass** die Vorrichtung dazu ausgelegt ist, jede Infusionspumpe (2-10) dazu anzusteuern, die Steuerungsdaten mittels jeweils einer ihnen zugeordneten Steuereinheit zu verarbeiten und direkt an mindestens eine der weiteren Infusionspumpen (2-10) zu übertragen.

5. Vorrichtung nach Anspruch 3 oder 4, **dadurch gekennzeichnet, dass** die Vorrichtung dazu ausgelegt ist, dass die Infusion von mindestens einer der Infusionspumpen (2-10) immer aktiviert ist.

6. Vorrichtung nach einem der Ansprüche 3 bis 5, **dadurch gekennzeichnet, dass** die Vorrichtung dazu ausgelegt ist, dass die Infusion sämtlicher Infusionspumpen (2-10) zeitgleich aktiviert und/oder deaktiviert werden.

7. Vorrichtung nach Anspruch 4, **dadurch gekennzeichnet, dass** die Vorrichtung dazu ausgelegt ist, dass mittels Betätigungselementen an den Steuereinheiten mindestens ein Steuervorgang zur Steuerung der Aktivierung und Deaktivierung der verschiedenen Infusionen für mindestens zwei Infusionspumpen eingegeben wird.

8. Vorrichtung nach Anspruch 7, **dadurch gekennzeichnet, dass** die Vorrichtung dazu ausgelegt ist, dass der Steuervorgang die gleichzeitige Aktivierung und/oder Deaktivierung und/oder eine Unterbrechung der Infusion durch die mindestens zwei Infusionspumpen (2-10) bewirkt.

9. Vorrichtung nach Anspruch 7 oder 8, **dadurch gekennzeichnet, dass** die Vorrichtung dazu ausgelegt ist, dass durch die Auswahl eines Therapievorganges, dem ein Steuervorgang zugeordnet ist, die dem Therapievorgang zuordbaren Infusionspumpen (2-10) selbsttätig ausgewählt oder einem Benutzer zur Auswahl angezeigt werden.

10. Vorrichtung nach einem der Ansprüche 4 bis 9, **dadurch gekennzeichnet, dass** mindestens eine der Infusionspumpen (2-10) ein Verdünnungsmittel zusätzlich zu einem Arzneiwirkstoff, der mittels einer weiteren Infusionspumpe zugeführt wird, zuführt.

11. Vorrichtung nach Anspruch 10, **dadurch gekennzeichnet, dass** die Vorrichtung dazu ausgelegt ist, dass die Steuerungsdaten eine Deaktivierung der Infusion des Verdünnungsmittels unter Beibehaltung der Aktivierung des Arzneiwirkstoffes bewirken.

## Claims

1. Device for controlling a plurality of infusion pumps (2-10) comprising an infusion pump carrier with a column (11) on which the plurality of infusion pumps is arranged, wherein each infusion pump (2-10) is assigned a respective infusion substance which can be supplied to a living being as an infusion at a predeterminable infusion rate within a predeterminable period of time assigned to it,
wherein the device is configured to automatically exchange previously determined control data for time-coordinated activation and deactivation by means of control units between the infusion pumps (2-10) without the interposition of operating personnel, wherein each infusion pump (2-10) has one of the control units which processes the control data and transmits it directly to control units of the further infusion pumps (2-10), and
wherein the device is configured such that an automatic transfer of control data to one or more preselected infusion pumps takes place as soon as the period of time of the supply of a first infusion from the infusion pump sending the control data is finished and a second infusion from the further infusion pump receiving the control data is to be administered to the patient by activating it,
**characterized in that**
the device is configured such that measured or estimated physiological data can be entered into the control units and continuously received during therapy in order to automatically adjust infusion rates, the periods of individual infusions, or the times of activation and deactivation by means of predetermined program steps.

2. Device according to claim 1, **characterized by** actuating elements at the infusion pumps (2-10) for the input of control commands, for the selection of control processes, for the selection of infusion pumps (2-10) involved in the control operations, and/or for the input of physiological data of the living being.

3. Device according to claim 1 or 2, **characterized by** a display device at each infusion pump and/or a central display device for displaying the control data, measured or estimated physiological data of the living being, the times of activation and deactivation of the infusion pumps, the periods of time of infusion, and/or the infusion rates.

4. Device according to claim 3, **characterized in that** the device is configured to drive each infusion pump (2-10) in order to process the control data by means of a control unit assigned to each of them and to transmit it directly to at least one of the further infusion pumps (2-10).

5. Device according to claim 3 or 4, **characterized in that** the device is configured such that the infusion from at least one of the infusion pumps (2-10) is always activated.

6. Device according to one of claims 3 to 5, **characterized in that** the device is configured such that the infusions of all infusion pumps (2-10) are activated and/or deactivated simultaneously.

7. Device according to claim 4, **characterized in that** the device is configured such that by means of actuating elements at the control units, at least one control process for controlling the activation and deactivation of the different infusions is input for at least two infusion pumps.

8. Device according to claim 7, **characterized in that** the device is configured such that the control process causes the simultaneous activation and/or deactivation and/or interruption of infusion by the at least two infusion pumps (2-10).

9. Device according to claim 7 or 8, **characterized in that** the device is designed such that, by selecting a therapy process to which a control process is assigned, the infusion pumps (2-10) which can be assigned to the therapy process are automatically selected or displayed to a user for selection.

10. Device according to one of claims 4 to 9, **characterized in that** at least one of the infusion pumps (2-10) supplies a diluent in addition to an active pharmaceutical agent which is supplied by means of a further infusion pump.

11. Device according to claim 10, **characterized in that** the device is configured such that the control data causes the deactivation of the infusion of the diluent while maintaining the activation of the active pharmaceutical agent.

## Revendications

1. Dispositif pour commander une majorité de pompes à perfusion (2-10), avec un support de pompe à perfusion avec une colonne au (11) au niveau de laquelle la pluralité de pompes à perfusion est agencée, dans lequel à chaque pompe à perfusion (2-10) est respectivement attribuée une substance de perfusion qui peut être délivrée à un être vivant sous forme de perfusion à un taux de perfusion pouvant être prédéterminé au sein d'une période de temps pouvant être prédéterminée qui lui est attribuée,
dans lequel le dispositif est conçu pour échanger des données de commande précédemment établies pour une activation et une désactivation coordonnées l'une avec l'autre dans le temps automatiquement entre les pompes à perfusion (2-10) sans interposition de l'opérateur au moyen d'unités de commande, dans lequel chaque pompe à perfusion (2-10) présente une des unités de commande laquelle traite et transmet les données de commande directement aux unités de commande des autres pompes à perfusion (2-10), et
dans lequel le dispositif est conçu de telle manière qu'une transmission automatique des données de commande à une ou plusieurs pompes à perfusion présélectionnées se produit dès que la période de temps de délivrance d'une première perfusion de la pompe à perfusion transmettant les données de commande est terminée et qu'une seconde perfusion de la pompe à perfusion supplémentaire recevant les données de commande doit être administrée au patient par son activation, **caractérisé en ce que** le dispositif est conçu de telle manière des données physiologiques mesurées ou estimées puissent être entrées dans les unités de commande et reçues en continu pendant une thérapie afin de modifier automatiquement des taux de perfusion, les périodes de temps de perfusions individuelles ou les moments d'activation et de désactivation au moyen d'étapes de programme prédéterminées.

2. Dispositif selon la revendication 1, **caractérisé par** des éléments d'actionnement sur les pompes à perfusion (2-10) pour la saisie d'ordres de commande, pour la sélection des opérations de commande, pour la sélection de pompes à perfusion impliquées dans les opérations de commande (2-10) et/ou pour la saisie de données physiologiques de l'être vivant.

3. Dispositif selon la revendication 1 ou 2, **caractérisé par** un appareil d'affichage sur chaque pompe à perfusion et/ou un appareil d'affichage central pour l'affichage des données de commande, des données physiologiques mesurées ou estimées de l'être vivant, des moments d'activation et de désactivation des pompes à perfusion, des périodes de temps de perfusion et/ou des taux de perfusion.

4. Dispositif selon la revendication 3, **caractérisé en ce que** le dispositif est conçu pour viser chaque pompe à perfusion (2-10) afin de traiter les données de commande au moyen respectivement d'une unité de commande attribuée à celle-ci et de les transmettre directement à au moins une des autres pompes à perfusion (2-10).

5. Dispositif selon la revendication 3 ou 4, **caractérisé en ce que** le dispositif est conçu de sorte que la perfusion d'au moins une des pompes à perfusion (2-10) soit toujours activée.

6. Dispositif selon la revendication 3 à 5, **caractérisé en ce que** le dispositif est conçu de sorte que la perfusion de l'ensemble des pompes à perfusion (2-10) soit activée et/ou désactivée simultanément.

7. Dispositif selon la revendication 4, **caractérisé en ce que** le dispositif est conçu de sorte qu'une opération de commande est saisie pour commander l'activation et la désactivation des différentes perfusions pour au moins deux pompes à perfusion au moyen d'éléments d'actionnement au niveau des unités de commande.

8. Dispositif selon la revendication 7, **caractérisé en ce que** le dispositif est conçu de sorte que l'opération de commande entraîne l'activation et/ou la désactivation simultanées et/ou une interruption de la perfusion par les au moins deux pompes à perfusion (2-10).

9. Dispositif selon la revendication 7 ou 8, **caractérisé en ce que** le dispositif est conçu de sorte que, par la sélection d'une opération de thérapie à laquelle une opération de commande est attribuée, les pompes à perfusion (2-10) qui peuvent être attribuées à l'opération de thérapie sont automatiquement sélectionnées ou affichées à un utilisateur pour la sélection.

10. Dispositif selon l'une quelconque des revendications 4 à 9, **caractérisé en ce qu'**au moins une des pompes à perfusion (2-10) délivre un agent diluant en plus d'un principe actif médicamenteux qui est délivré au moyen d'une autre pompe à perfusion.

11. Dispositif selon la revendication 10, **caractérisé en ce que** le dispositif est conçu de sorte que les données de commande provoquent la désactivation de la perfusion de l'agent diluant, tout en maintenant l'activation du principe actif médicamenteux.
